**Europäisches Patentamt**

**European Patent Office**

**Office européen des brevets**

(19)

(11) Veröffentlichungsnummer: **0 002 274**
**A1**

(12) **EUROPÄISCHE PATENTANMELDUNG**

(21) Anmeldenummer: **78101516.9**

(22) Anmeldetag: **01.12.78**

(51) Int. Cl.²: **A 61 G 1/00**
**A 61 F 7/00**

(30) Priorität: **03.12.77 DE 2753911**

(43) Veröffentlichungstag der Anmeldung:
**13.06.79 Patentblatt 79/12**

(84) Benannte Vertragsstaaten:
**BE CH FR GB IT LU NL SE**

(71) Anmelder: **Hess, Helmut, Dr.**
**Dieburger-Strasse 94**
**D-6100 Darmstadt(DE)**

(84) Benannte Vertragsstaaten:
**BE CH FR GB IT LU NL SE**

(71) Anmelder: **Hess, Helmut, Jun.**
**Dieburger-Strasse 94**
**D-6100 Darmstadt(DE)**

(84) Benannte Vertragsstaaten:
**BE CH FR GB IT LU NL SE**

(72) Erfinder: **Hess, Helmut, Dr.**
**Dieburger-Strasse 94**
**D-6100 Darmstadt(DE)**

(72) Erfinder: **Hess, Helmut, Jun.**
**Dieburger-Strasse 94**
**D-6100 Darmstadt(DE)**

(54) **Rettungskapsel.**

(57) Der Anmeldung liegt die Aufgabe zugrunde, eine Vorrichtung zur Bergung und Behandlung von Unfallopfern mit Unterkühlungserscheinungen zu schaffen, die unmittelbar am Unfallort einsetzbar ist, zumindest aber in dessen Nähe gebracht werden kann, und die speziell für die Wiedererwärmung konzipiert ist.

Zur Lösung dieser Aufgabe wird eine aus einer unteren Halbschale (1) und einer oberen Halbschale (2) zusammensetzbare geschlossene Rettungskapsel vorgeschlagen, in deren unterer Halbschale (1) eine heizbare Unterlage angeordnet ist und deren obere Halbschale (2) verschließbare Behandlungsöffnungen aufweist. Die Halbschalen (1, 2) können aus im Abstand zueinander angeordneten glatten äußeren und inneren Wandungen mit dazwischen befindlichem Isoliermaterial bestehen. In der unteren Halbschale (1) können Auflageflächen zur Lagerung der Arme und Beine in erhöhter Position vorgesehen sein. Die heizbare Unterlage kann mit einer chemischen, bei Zufuhr von Flüssigkeit exotherm reagierenden Substanz gefüllt sein.

·/...

EP 0 002 274 A1

Croydon Printing Company Ltd.

Fig. 2

Dr.med.Helmut H E S S
Facharzt f.Frauenkrankheiten
&
Helmut H E S S jun.
cand.med.

Dieburger-Str.94
6100 D a r m s t a d t

Darmstadt, 29.11.1977

## RETTUNGSKAPSEL

Die Erfindung betrifft eine Vorrichtung zur Bergung und
Behandlung von Unfallopfern mit Unterkühlungserscheinungen.

Bei Unfällen sind Menschen häufig über einen längeren Zeitraum sehr niedrigen Temperaturen ausgesetzt, ohne irgendwelche Schutzmaßnahmen dagegen treffen zu können. So werden
beispielsweise Schiffbrüchige, Lawinenopfer oder auch Opfer
nach Verkehrsunfällen mit lebensbedrohenden Unterkühlungserscheinungen aufgefunden. In derartigen Fällen muß zur
Rettung eine rasche Wiedererwärmung des Opfers vorgenommen
werden, um irreparable Schädigungen oder gar den Tod zu
vermeiden.

Neben unzureichenden vorläufigen Maßnahmen wird die Wiedererwärmung seit langem unverändert in der Weise durchgeführt,
daß das Unfallopfer schnellstmöglich ein heißes Vollbad erhält. Ein derartiges Vorgehen setzt jedoch entsprechende Einrichtungen (Badewanne, Heißwasserbereitung) voraus, die vielfach nur weit entfernt vom Unfallort zur Verfügung stehen.
Die Rettungschancen sind also davon abhängig, wie schnell
das Unfallopfer zu derartigen Einrichtungen transportiert
werden kann. Darin besteht ein erheblicher Nachteil der bisher
üblichen Wiedererwärmung von Unfallopfern mit Unterkühlungserscheinungen.

Hinzu kommt, daß die erwähnten Einrichtungen nur als ein Behelf angesehen werden können. Bei schweren Unterkühlungen muß nämlich darauf geachtet werden, daß die Wiedererwärmung zunächst im Kopf- und Körperbereich durchgeführt werden kann, daß also Arme und Beine zunächst kalt bleiben oder gar gleichzeitig gekühlt werden, um eine Kollapsgefahr zu vermeiden. Eine derartige partielle Wiedererwärmung ist in üblichen Badewannen nur schlecht zu realisieren, weil die Extremitäten aus dem heißen Wasser herausgehalten werden müssen.

Der Erfindung liegt die Aufgabe zugrunde, die geschilderten Nachteile zu vermeiden, d.h. eine Vorrichtung zur Bergung und Behandlung von Unfallopfern mit Unterkühlungserscheinungen zu schaffen, die unmittelbar am Unfallort einsetzbar ist, zumindest aber in dessen Nähe gebracht werden kann, und die speziell für die geschilderte Wiedererwärmung konzipiert ist.

Zur Lösung dieser Aufgabe wird eine aus einer unteren und einer oberen Halbschale zusammensetzbare geschlossene Rettungskapsel vorgeschlagen, in deren unterer Halbschale eine heizbare Unterlage angeordnet ist und deren obere Halbschale verschließbare Behandlungsöffnungen aufweist.

In weiterer Ausbildung des Erfindungsgedankens ist vorgesehen, daß die Halbschalen aus Kunststoff geformt sind. Sie bestehen zweckmäßigerweise aus im Abstand zueinander angeordneten glatten äußeren und inneren Wandungen, zwischen denen sich Isoliermaterial befindet. Gemäß einer vorteilhaften Ausführungsform bestehen die Wandungen aus glasfaserverstärktem Kunststoff, wobei der Raum zwischen ihnen mit Styropor ausgefüllt ist. Nach einer anderen Ausführungsform sind die Halbschalen einstückig in Sandwich-Bauweise gefertigt, wobei die Wandungen über dazwischen befindlichem Hartschaum miteinander verbunden sind.

Zur Lagerung der Arme und Beine in erhöhter Position sind
erfindungsgemäß in der unteren Halbschale Auflageflächen
vorgesehen.

Eine wesentliche Verbesserung erfährt die Vorrichtung dadurch, daß die heizbare Unterlage mit einer chemischen
Substanz gefüllt werden kann, die bei Zufuhr von Flüssigkeit exoterm
reagiert. Ferner ist vorgesehen, daß die Unterlage im Bereich der Auflageflächen wahlweise nicht oder unabhängig von
den übrigen Bereichen heizbar ist.

Zur weiteren Ausbildung der Rettungskapsel ist vorgesehen,
daß sie mit Einrichtungen zum Tragen und/oder zum Fahren
und/oder zum Aufhängen versehen ist. Die Behandlungsöffnungen
sollen mit Klappen verschließbar sein, die wahlweise mit
Fenstern ausgestattet sind. Ferner ist es zweckmäßig, wenn
die Rettungskapsel weiteres Zubehör, wie eine heizbare Decke,
eine Alu-Rettungsfolie, Blutkonserven, eine Sauerstoffflasche
sowie Stetoskop, Termometer und dergleichen enthält. Schließlich weist die Rettungskapsel erfindungsgemäß Anschlüsse
und Regeleinrichtungen für die Zufuhr von erwärmtem Wasser
in die heizbare Unterlage und/oder für die Zufuhr elektrischer Energie sowie für die Regelung der Beheizung auf.

Mit der vorgeschlagenen Rettungskapsel ist eine Bergung und
Behandlung von Unfallopfern mit Unterkühlungserscheinungen
wesentlich schneller und effektiver durchzuführen als mit
der bisher üblichen Wiedererwärmung im heißen Vollbad. Die
Rettungskapsel ist nicht nur leicht unmittelbar an den Unfallort oder in dessen Nähe zu transportieren, wo die Behandlung sofort beginnen kann. Durch die "netzunabhängige"
Beheizung mittels exoterm reagierender chemischer Stoffe,
wie z.B. CaO, ist eine mobile Behandlungsstation geschaffen,
die den bisher gebräuchlichen Einrichtungen weit überlegen ist.

Hinzu kommt, daß mit der speziellen Ausgestaltung und Ausrüstung der Rettungskapsel eine Vorrichtung geschaffen wurde, die nicht mehr nur als Provisorium für die Wiedererwärmung unterkühlter Unfallopfer angesehen werden kann. Sie ist aufgrund der verwendeten Materialien und der angepaßten konstruktiven Ausbildung auf Schiffen, Fahrzeugen oder Bergstationen leicht aufzubewahren und jederzeit einsetzbar.

Für die "netzunabhängige" Beheizung der Unterlage, kann beispielsweise eine Mischung aus 90 bis 95 Teilen Eisenpulver und 5 bis 10 Teilen Kaliumpersulfat oder eine Mischung aus je einem Teil Magnesiumpulver und Kieselgur mit drei Teilen Zinkammoniumchlorid verwendet werden. Eine mit derartigen Pulvermischungen gefüllte Unterlage, kann am Unfallort leicht mit einer geringen Wassermenge aktiviert werden und gibt dann für längere Zeit die erforderliche Wärme ab.

Zur "netzunabhängigen" Beheizung können alternativ auch Sonnenkollektoren angewendet werden, die mit fortschreitender Entwicklung auch bei ungünstigen Einstrahlungsverhältnissen ausreichend Nutzwärme oder elektrischen Strom erzeugen können.

✗

Nach Bergung eines Unfallopfers und Transport zu Versorgungsstationen mit Netzanschluß oder eigener Energieerzeugung, kann die Rettungskapsel an die Warmwasserversorgung oder an das elektrische Netz angeschlossen werden. Dazu ist entweder in der heizbaren Unterlage oder aber im Isoliermaterial zwischen innerer und äußerer Schalenwandung ein Rohrsystem oder ein Heizdraht verlegt. Über entsprechende Anschlüsse und Regeleinrichtungen an der Rettungskapsel ist so eine vollständige Wiedererwärmung des Unfallopfers möglich.

Darüber hinaus ist die erfindungsgemäße Rettungskapsel auch bei der Hyperthermiebehandlung Krebskranker einsetzbar, wobei die eingebauten Beheizungseinrichtungen ggfs. weiter

---

✗ Eine weitere Möglichkeit netzunabhängiger Beheizung ist z.B. auch die Anwendung einer an sich bekannten Auto-Stand-Heizungsanlage, die mit Benzin oder Dieseloel betrieben werden kann.

unterteilt und auf eine gezielte örtliche Erwärmung bzw.
auf die erforderlichen unterschiedlichen Temperaturen eingerichtet werden können.

Weitere Einzelheiten und Vorteile der erfindungsgemäßen
Rettungskapsel werden anhand des in den Figuren dargestellten
Ausführungsbeispiels näher erläutert:

Figur 1 zeigt eine Draufsicht auf die Rettungskapsel.

Figur 2 zeigt eine Seitenansicht der Rettungskapsel.

Figur 3 zeigt eine Ansicht des fußseitigen Endes der
Rettungskapsel.

Figur 4 zeigt einen Teillängsschnitt sowie einen Querschnitt
durch die untere Halbschale.

Figur 5 zeigt einen Querschnitt durch den Beinteil der geschlossenen Rettungskapsel.

Figur 6 zeigt eine Draufsicht der heizbaren Unterlage.

Figur 7 zeigt die Stapelmöglichkeit der Halbschalen.

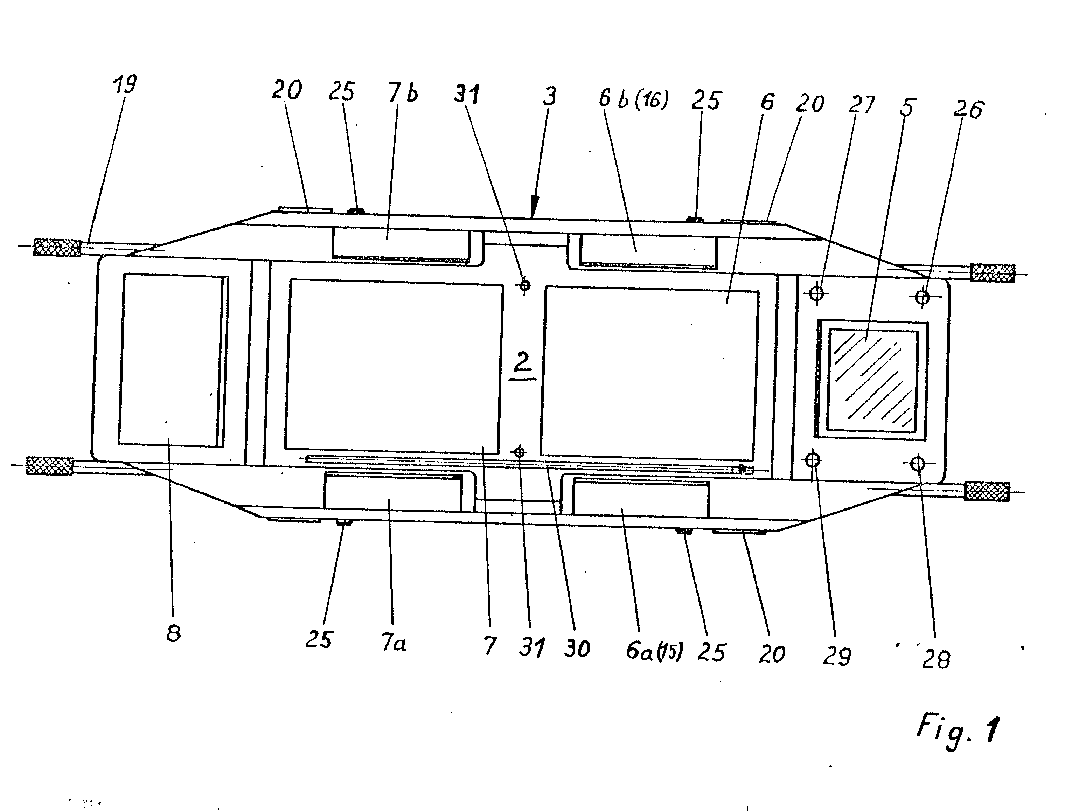

In der Draufsicht auf die Rettungskapsel 3 gemäß Figur 1,
sind im wesentlichen die Einzelheiten der oberen Halbschale 2
zu sehen. So die vier großen Behandlungsöffnungen für den
Kopf 5, den oberen Körperteil 6, den unteren Körperteil 7
und die Füße 8. Außerdem sind seitlich neben den Behandlungsöffnungen 6 und 7 je zwei kleinere Öffnungen 6a,b und 7a,b
vorgesehen. Unter den Behandlungsöffnungen 6 a,b befinden sich
in der unteren Halbschle die nicht dargestellten erhöhten
Auflageflächen 15,16 für die Arme.

Zum Transport der Rettungskapsel sind einmal vier einschiebbare Tragholme 19 sowie vier einklappbare Traggriffe 20 angeordnet. Die obere und untere Halbschale
können durch Verschlüsse 25 miteinander fest verbunden
werden. Ferner sind im Kopfbereich kleinere Öffnungen für
das Einstecken eines Thermometers 26, für die Sauerstoffzufuhr 27, für ein Überdruckventil 28 und für den Luftauslaß 29 vorgesehen. Außerdem ist an der oberen Halbschale
ein Infusionsständer 30 befestigt, der bei Bedarf in eine
der Öffnungen 31 eingesteckt werden kann.

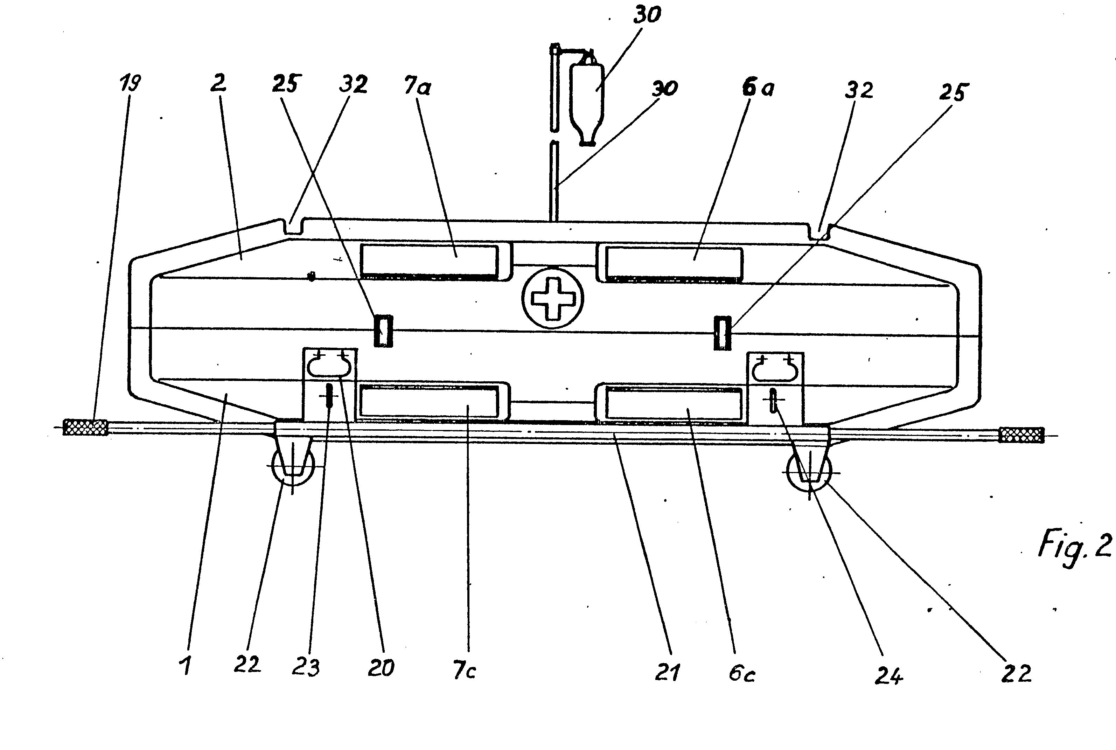

Figur 2 zeigt eine Seitenansicht der Rettungskapsel mit der
unteren Halbschale 1 und der oberen Halbschale 2, die durch
die Verschlüsse 25 fest miteinander verbindbar sind.
Symmetrisch zu den kleinen Behandlungsöffnungen 6a und 7a,
sind in der unteren Halbschale kleine Kammern 6 c und 7c
für Zubehör vorgesehen.

In den Rahmen 21, an dem die Räder 22 befestigt sind, können
die Tragholme 19 eingeschoben werden. Ferner sind an der
unteren Halbschale 1 die Traggriffe 20 sowie jeweils darunter
Ösen 23, 24 zu sehen, die zum Anschlagen von Seilen oder dergleichen beim Transport der Kapsel mittels Hubschrauber diene
Schließlich zeigt die Seitenansicht noch, wie der Infusionsständer 30 eingesteckt ist und eine Infusionsflasche 30a
trägt, sowie Ausnehmungen 32 in der oberen Halbschale 2, die
auch in der unteren Halbschale vorgesehen sind, um die beiden
Halbschalen auch mittels Gurten zusammenzuhalten.

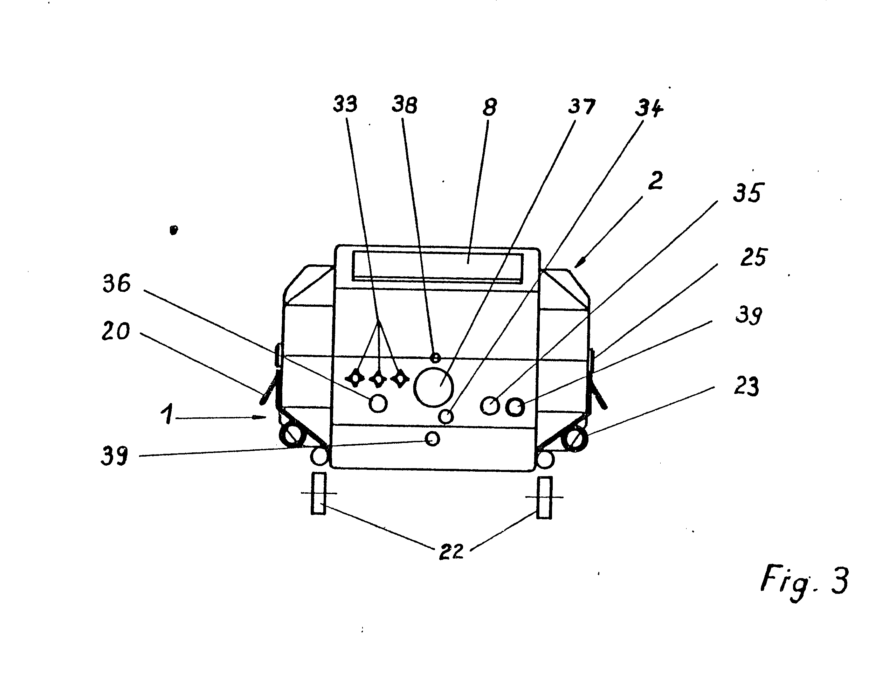

Figur 3 zeigt eine Ansicht des fußseitigen Endes der
Rettungskapsel, wiederum mit unterer und oberer Halbschale 1,
sowie den bereits erwähnten Einzelheiten, wie Räder 22,
Ösen 23, Traggriffen 20, Verschlüssen 25 und Behandlungsöffnung ö. An dem fußseitigen Ende sind die Anschlüsse für Kaltwasser 35, Warmwasser 36 und Strom 38 angeordnet.
Ferner die Mischventile 33 sowie eine Frischluftöffnung 34
und ein Wasserabfluß 39. Mit 37 ist ein Aufnahmefach bezeichnet, in dem beispielsweise eine Sauerstoffflasche
untergebracht werden kann.

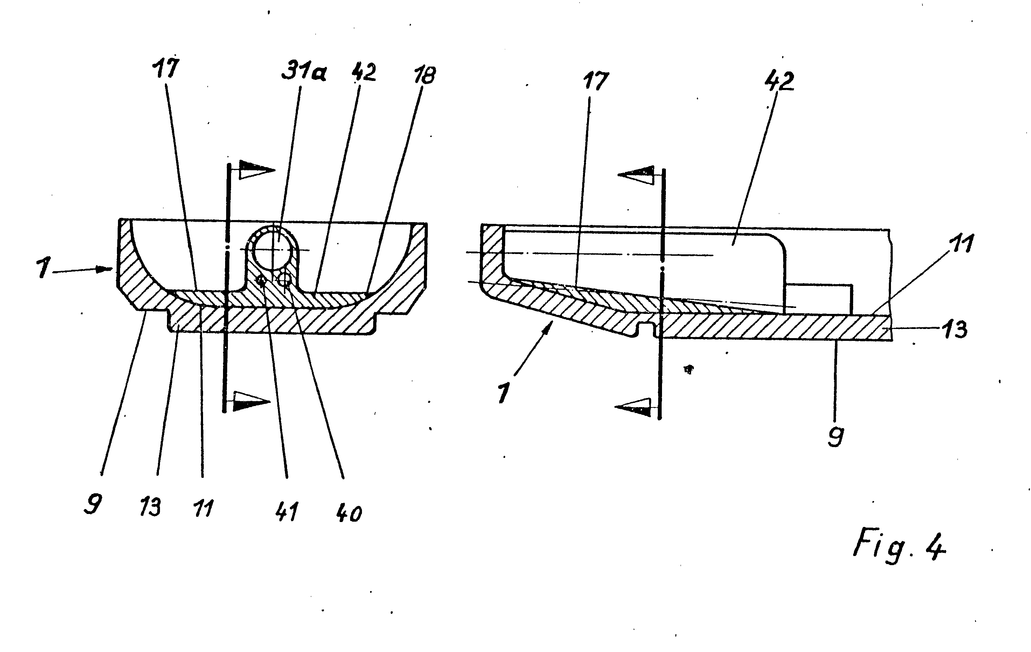

Figur 4 zeigt Einzelheiten des Schalenaufbaus in einem Teillängsschnitt und einem Querschnitt durch das fußseitige
Ende der unteren Halbschale 1. Die Halbschale besteht aus
einer äußeren Wandung 9 und einer inneren Wandung 10 aus
glasfaserverstärktem Kunststoff, zwischen denen sich Isolierstoff 13, beispielsweise Styropor, befindet. In die untere
Halbschale 1 ist fußseitig ein keilförmig ansteigendes Teil 42
eingelegt, das die erhöhte Auflage 17, 18 für die Beine
bildet und in der Mitte ein Aufnahmefach für beispielsweise
eine Infusionsflasche 31a enthält. Ferner ist in dem keilförmigen Teil 42 eine Leitung für die Luftzufuhr 40 und für
die Urinabfuhr 41 vorgesehen.

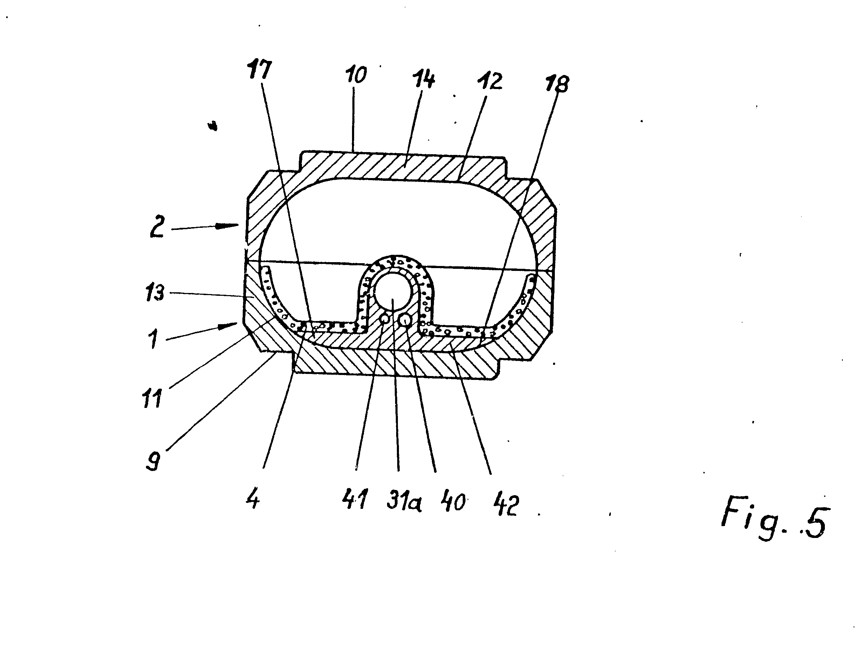

Figur 5 zeigt noch einmal einen Querschnitt durch die
Rettungskapsel im Beinbereich. Die obere Halbschale 2 ist
wie die untere Halbschale 1 aufgebaut und besitzt eine
innere 12 und eine äußere 10 Wandung mit dazwischen angeordnetem Isoliermaterial 14. In die untere Halbschale 1 ist
die heizbare Unterlage 4 eingelegt. Im übrigen entspricht die
Bezifferung der Figur 4.

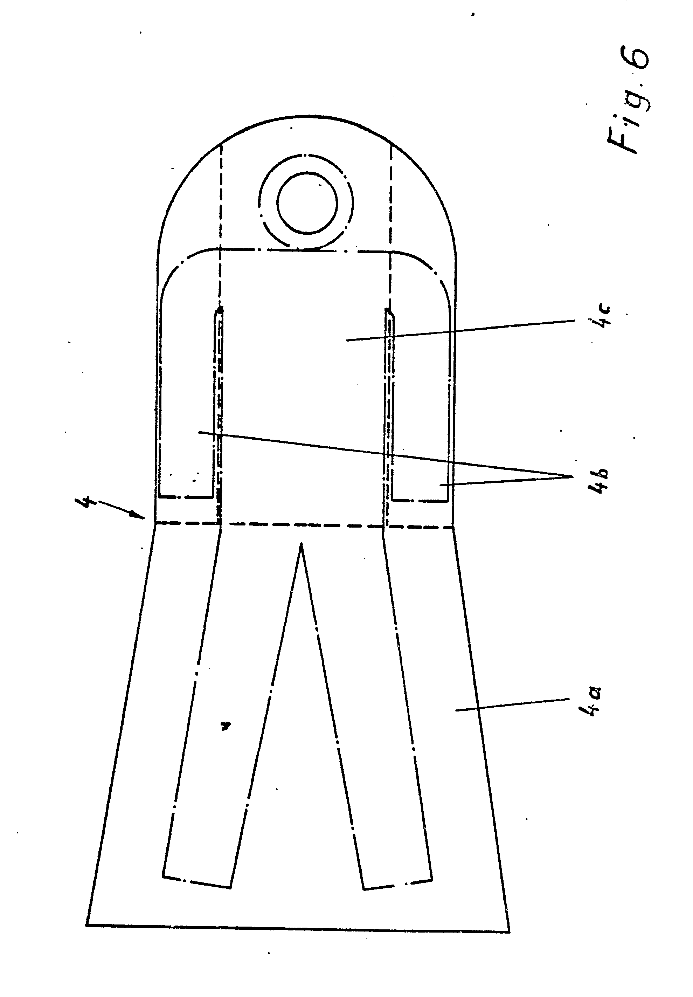

In Figur 6 ist die heizbare Unterlage 4 etwas schematisiert
dargestellt, wobei mit 4a, b und c die getrennt heizbaren
Bereiche für die Beine (a), die Arme (b) und den Rumpf mit
Kopf (c) bezeichnet sind.

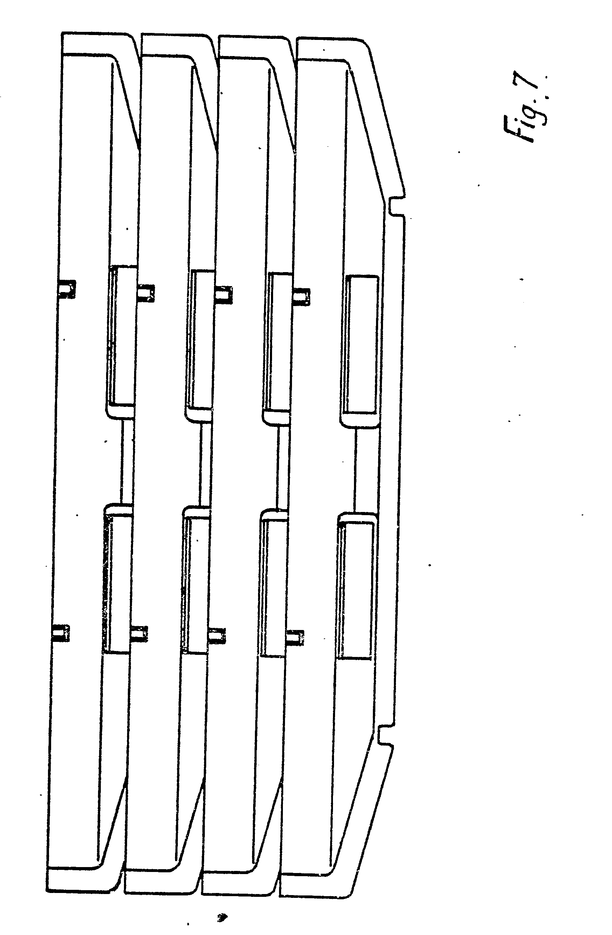

Figur 7 schließlich zeigt, wie die Halbschalen platzsparend
stapelbar sind, was insbesondere bei beengten Raumverhältnissen wie auf Schiffen wichtig ist.

## PATENTANSPRÜCHE

1. Vorrichtung zur Bergung und Behandlung von Unfallopfern mit Unterkühlungserscheinungen, gekennzeichnet
durch eine untere Halbschale (1) und eine obere Halbschale (2), die zu einer geschlossenen Rettungskapsel (3)
zusammensetzbar sind, durch eine in der unteren Halbschale (1) angeordnete heizbare Unterlage (4), sowie
durch verschließbare Behandlungsöffnungen (5,6,7,8)
in der oberen Halbschale (2).

2. Vorrichtung nach Anspruch 1, dadurch gekennzeichnet,
daß die Halbschalen (1,2) aus Kunststoff geformt sind.

3. Vorrichtung nach Anspruch 2, dadurch gekennzeichnet,
daß die Halbschalen (1,2) aus im Abstand zueinander
angeordneten glatten äußeren und inneren Wandungen
(9,10,11,12) bestehen und daß sich zwischen den äußeren
und inneren Wandungen (9,10,11,12) Isoliermaterial
(13,14) befindet.

4. Vorrichtung nach Anspruch 3, dadurch gekennzeichnet,
daß die Wandungen (9,10,11,12) aus glasfaserverstärktem
Kunststoff bestehen und daß der Raum zwischen ihnen mit
Styropor ausgefüllt ist.

5. Vorrichtung nach Anspruch 2, dadurch gekennzeichnet,
daß die Halbschalen einstückig in Sandwich-Bauweise gefertigt sind, wobei die Wandungen über dazwischen befindlichem Hartschaum miteinander verbunden sind.

6. Vorrichtung nach einem der Ansprüche 1 bis 5, dadurch
gekennzeichnet, daß in der unteren Halbschale (1) Auflageflächen (15,16,17,18) zur Lagerung der Arme und
Beine in erhöhter Position vorgesehen sind.

- 2 -

0002274

7. Vorrichtung nach einem der Ansprüche 1 bis 6, <u>dadurch gekennzeichnet</u>, daß die heizbare Unterlage (4) mit einer chemischen Substanz gefüllt ist, die bei Zufuhr von Flüssigkeit exoterm reagiert.

8. Vorrichtung nach Anspruch 7, <u>dadurch gekennzeichnet</u>, daß die Unterlage (4) im Bereich der Auflageflächen (15,16,17,18) wahlweise nicht oder unabhängig von den übrigen Bereichen heizbar ist.

9. Vorrichtung nach einem der Ansprüche 1 bis 8, <u>dadurch gekennzeichnet</u>, daß die Rettungskapsel (3) mit Einrichtungen zum Tragen (19,20) und/oder zum Fahren (21,22) und/oder zum Aufhängen (23,24) versehen ist.

10. Vorrichtung nach einem der Ansprüche 1 bis 9, dadurch <u>gekennzeichnet</u>, daß die Behandlungsöffnungen mit Klappen verschließbar sind, die wahlweise mit Fenstern ausgestattet sind.

11. Vorrichtung nach einem der Ansprüche 1 bis 10, <u>dadurch gekennzeichnet</u>, daß die Kapsel weiteres Zubehör wie eine heizbare Decke, eine Alu-Rettungsfolie, Blutkonserven, eine Sauerstoffflasche, sowie Stetoskop, Termometer und dergleichen enthält.

12. Vorrichtung nach einem der Ansprüche 1 bis 11, <u>dadurch gekennzeichnet</u>, daß die Rettungskapsel ferner Anschlüsse und Regeleinrichtungen für die Zufuhr von erwärmtem Wasser in die heizbare Unterlage und/oder für die Zufuhr elektrischer Energie sowie für die Regelung der Beheizung aufweist.

Fig. 1

Fig.2

2/7

0002274

Fig. 3

4/7

17    31a   42   18

1

9   13   11   41   40

17         42

1

11

13

9

Fig. 4

Fig. 5

0002274

Fig. 6

0002274

Fig. 7

**Europäisches Patentamt**

# EUROPÄISCHER RECHERCHENBERICHT

Nummer der Anmeldung

EP 78 101 516.9

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int.Cl.²) |
|---|---|---|---|
| – | <u>DE - U - 7 524 657</u> (COAL INDUSTRY) <br> * gesamtes Dokument* | 1,2, 4,9, 11 | A 61 G 1/00 <br> A 61 F 7/00 |
| – | <u>DE - A - 2 259 894</u> (INFRANOR) <br> * Ansprüche 1, 8, 10, 15, 16; Fig. 1, 2 * | 1,2, 9,10 | |
| – | <u>DE - U - 1 896 209</u> (J. EHLER) <br> * Anspruch 8 * | 1,7 | **RECHERCHIERTE SACHGEBIETE (Int. Cl.²)** |
| – | <u>DE - U - 6 608 268</u> (VEB ELEKTRO-GERÄTEWERK SUHL u.a.) <br> * Anspruch 1 * | 1,12 | A 61 F 7/00 <br> A 61 G 1/00 <br> A 61 G 11/00 |
| P | <u>DE - U - 7 736 9.?</u> (H. HESS u.a.) <br> * gesamtes Dokument * | 1-12 | |
| A | <u>DE - B - 2 241 937</u> (DRÄGERWERK) <br> * gesamtes Dokument * | | |
| A | <u>DE - U - 7 526 211</u> (K. SEMM) <br> * gesamtes Dokument * | | |
| | ./.. | | |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

[X] Der vorliegende Recherchenbericht wu : für alle Patentansprüche erstellt.

| Recherchenort | Abschlu. i ium der Recherche | Prüfer |
|---|---|---|
| Berlin | 1-02-1979 | DROPMANN |

EPA form 1503.1 06.78

| | **EINSCHLÄGIGE DOKUMENTE** | | **KLASSIFIKATION DER ANMELDUNG (Int.Cl.³)** |
|---|---|---|---|
| Kategorie | Kennzeichnung des Dokuments mit Angabe, soweit erforderlich, der maßgeblichen Teile | betrifft Anspruch | |
| A | <u>DE - B - 2 003 137</u> (SIEMENS)<br>* gesamtes Dokument *<br><br>-- | | |
| A | <u>DE - A - 2 112 505</u> (NEDERLANDSE ORGANISATIE VOOR TOEGEPAST NATUUR-WETENSCHAPPELIJK ONDERZOEK)<br>* gesamtes Dokument *<br><br>-- | | |
| | | | **RECHERCHIERTE SACHGEBIETE (Int. Cl.³)** |

EPA Form 1503.2 06.78